# FASCICULE DE BREVET EUROPEEN

(11) **EP 1 052 995 B1**
(45) Date de publication et mention de la délivrance du brevet: **20.11.2002**
(21) Numéro de dépôt: 99901679.3
(22) Date de dépôt: 02.02.1999
(51) Int. Cl.: A61K 31/70, A61K 9/00, A61K 47/40, A61K 47/24, A61K 47/26

(54) **COMPOSITION PHARMACEUTIQUE POUR L'ADMINISTRATION PAR VOIE NASALE DE THIOCOLCHICOSIDE**
PHARMAZEUTISCHE ZUSAMMENSETZUNG ZUR NASALEN VERABREICHUNG VON THIOCOLCHICOSID
PHARMACEUTICAL COMPOSITION FOR NASAL ADMINISTRATION OF THIOCOLCHICOSIDE

(30) Priorité: 05.02.1998 FR 9801328
(43) Date de publication de la demande: 22.11.2000
(73) Titulaire: SANOFI-SYNTHELABO, 75013 Paris (FR)
(72) Inventeur: COLOMBO, Paolo, I-43100 Parma (IT); SANTI, Patrizia, I-43039 Salsomaggiore Terme (IT); ARTUSI, Mariella, I-43100 Parma (IT)
(74) Mandataire: Thouret-Lemaitre, Elisabeth
(86) Numéro de dépôt international: FR9900204
(87) Numéro de publication internationale: WO99039717

(56) Documents cités:
- EP-A- 0 733 357
- WO-A-97/01570
- WO-A-97/47577
- WO-A-98/36751
- GB-A- 1 345 991
- "MARTINDALE, The Extra Pharmacopoeia, 29th edition" , THE PHARMACEUTICAL PRESS , LONDON XP002105366 voir page 1240, colonne 2, alinéa 13324-V

## Description

La présente invention est relative à une composition pharmaceutique pour l'administration nasale de thiocolchicoside.

Le thiocolchicoside peut être utilisé dans le traitement d'appoint des contractures musculaires douloureuses. Il est habituellement administré par voie orale (WO-A-9836 751 et MARTINDALE, The Extra Pharmacopoeia, 29^{th} edition, the Pharmaceutical Press, LONDON, p. 1240, ∮ 13324-V), sous forme de comprimés, d'ampoules ou de gélules ou par voie parentérale (par injection ou localement par application de pommade). Des études pharmacocinétiques sur l'homme ont montré que la durée de la demi-vie est de une à deux heures et que la biodisponibilité du thiocolchicoside, après administration par voie orale est de 25% environ par rapport à la voie intramusculaire et varie considérablement d'un individu à l'autre. Ce phénomène peut être attribué, principalement à un effet de premier passage hépatique et au cycle entéro-hépatique.

Les compositions pharmaceutiques, objets de l'invention, permettent de remédier à ces inconvénients. En effet, la muqueuse nasale, qui occupe une large surface (150 cm²), possède une riche vascularisation et une faible activité enzymatique. La muqueuse nasale se prête donc à l'absorption du principe actif, pourvu qu'elle soit maintenue en contact avec le thiocolchicoside pendant un délai suffisant, grâce à l'emploi d'une forme pharmaceutique appropriée.

Outre l'élimination de l'effet de premier passage hépatique et l'augmentation de la biodisponibilité, décrits ci-dessus, les autres avantages des compositions pharmaceutiques selon l'invention sont nombreux :
- l'administration du thiocolchicoside est rendue aisée et indolore,
- les compositions selon l'invention présentent une formulation très stable et nettement simplifiée, en particulier par rapport aux comprimés,
- enfin, les compositions selon l'invention permettent d'obtenir une concentration accrue de thiocolchicoside sur le site d'application, c'est à dire la muqueuse nasale, provoquée par une dissolution rapide du principe actif et une augmentation d'environ 30% de la solubilité du thiocolchicoside.

Les compositions selon l'invention sont constituées d'une poudre, dite poudre nasale, comprenant le principe actif et un ou plusieurs excipients pharmaceutiquement acceptables. Par poudre nasale on entend une substance solide finement broyée ou pilée caractérisée en ce qu'elle est hydrosoluble et rapidement soluble *in situ* dans les liquides physiologiques de la muqueuse nasale. L'application de cette poudre sur la muqueuse nasale peut requérir l'utilisation d'un pulvérisateur nasal approprié.

Certains excipients utilisés pour les compositions selon l'invention sont les substances d'origine naturelle ou synthétique, capables de se dissoudre dans une petite quantité de liquide physiologique de la muqueuse nasale et de favoriser une augmentation de la concentration en thiocolchicoside dans la solution obtenue sur le site d'application. Parmi ces excipients on peut notamment citer le mannitol, le sorbitol, le lactose, la cyclodextrine, l'amidon soluble, la gélatine, les celluloses et leurs dérivés, la polyvinylpyrrolidone, la pectine, l'alginate de sodium, le polyéthylèneglycol, le polyméthacrylate et le dextrane.

Par cyclodextrine, on entend les différents types de cyclodextrine et leurs dérivés tels que, par exemple, la β-cyclodextrine, la diméthylcyclodextrine, la 2-hydroxyéthylcyclodextrine, la 2-hydroxypropylcyclodextrine, la 3-hydroxypropylcyclodextrine et la triméthylcyclodextrine.

Les compositions selon l'invention peuvent comprendre du thiocolchicoside de 2 à 10 mg par unité. De préférence, la quantité de thiocolchicoside varie de 2 à 8 mg par unité.

Les compositions pharmaceutiques selon l'invention peuvent être à libération immédiate ou prolongée. Ces deux types de compositions, décrites ci-après, font partie de l'invention.

Les compositions à libération immédiate permettent d'obtenir un effet analogue à celui pouvant être obtenu par injection. Lorsque la poudre est appliquée sur la muqueuse nasale, le principe actif se dissout très rapidement dans les liquides physiologiques présents. Une solution très concentrée en thiocolchicoside se forme sur le site d'application, favorisant son absorption rapide.

Ces compositions à libération immédiate peuvent avantageusement être constituées de thiocolchicoside, d'une cyclodextrine et éventuellement, d'un ou plusieurs autres excipients. A titre de ce ou de ces autres excipients, on peut utiliser, par exemple, ceux cités précédemment, page 2, ligne 21 à 26 de la description.
La β-cyclodextrine est la cyclodextrine préférée. Les autres dérivés de cyclodextrine, cités précédemment, peuvent également être utilisés.
Le mélange de thiocolchicoside avec la cyclodextrine peut donner lieu à une dissolution complète du principe actif en 2 minutes environ avec une concentration saturée d'environ 30% supérieure à celle obtenue avec le thiocolchicoside seul. La cyclodextrine joue le rôle de dispersant, augmente la vitesse de dissolution de la poudre nasale et la solubilité de thiocolchicoside, et agit, de plus, comme un promoteur d'absorption, ce qui permet d'accélérer la pénétration du principe actif à travers la muqueuse nasale.
En masse, le pourcentage en thiocolchicoside varie de 5 à 40%, en cyclodextrine de 30 à 90% et en excipients de 0 à 65%.
Cette poudre nasale peut être préparée en mélangeant à sec les composants suivi de leur tamisage.

Les compositions à libération prolongée permettent, quant à elles, d'obtenir un effet plus durable, utile notamment lors d'une cure de longue durée.

La poudre de ces compositions à libération prolongée est caractérisée par la présence d'un phospholipide, dans lequel se dissout le principe actif et d'un ou plusieurs autres excipients solubles dans les liquides physiologiques de la muqueuse nasale. Lorsque la poudre est appliquée sur la muqueuse nasale, elle s'hydrate et donne lieu à une dispersion de vésicules phospholipidiques (liposomes) contenant le principe actif. Ces liposomes jouent un rôle de réservoir, favorisant l'accumulation de thiocolchicoside dans la muqueuse nasale. La dispersion créée *in situ* ralentit l'élimination du mucus et permet ainsi un temps de contact plus long entre le principe actif et la site d'absorption, par rapport à une composition à libération immédiate.
On peut utiliser des phosphlipides tels que : céphaline, phosphatidylsérine, sphingomyéline, phosphatidylinositol, phosphatidyléthanolamine et lécithine. La lécithine est le phospholipide préféré.
A titre d'excipient, on peut utiliser, par exemple, ceux cités précédemment, page 2, ligne 21 à 26 de la description. En masse, le pourcentage en thiocolchicoside varie de 5 à 40%, en phospholipide de 6 à 15% et en excipient inerte soluble dans les liquides physiologiques de la muqueuse nasale de 45 à 89%.
Cette poudre nasale peut être préparée par mélange, granulation directe puis tamisage ou au moyen d'un atomiseur.

L'application de ces deux types de compositions selon l'invention peut être réalisée au moyen d'un pulvérisateur nasal adapté.

Ces compositions pharmaceutiques, objets de l'invention, testés chez les volontaires sains, ont montré une excellente tolérance locale. Elles n'ont pas provoqué de phénomène d'irritation locale ou de malaise.

Les exemples suivants illustrent l'invention. Les compositions y sont décrites, ainsi que les méthodes de préparation des poudres constitutives des compositions de l'invention.

### Exemple 1 : Composition pharmaceutique à libération immédiate

Préparation de 20 g de poudre nasale :

| | | |
|---|---|---|
| Composants | thiocolchicoside | 2 g |
| | β-cyclodextrine | 18 g |

Le thiocolchicoside et la β-cyclodextrine ont été mélangés dans un mortier automatique pendant une heure.
Le mélange a été tamisé à travers des tamis de 250 µm afin d'éliminer la fraction de poudre plus grossière.

### Exemple 2 : Composition pharmaceutique à libération prolongée

Préparation de 20 g de poudre nasale :

| | | |
|---|---|---|
| Composants | thiocolchicoside | 2 g |
| | mannitol | 16,66 g |
| | lécithine | 1,34 g |

Selon une première méthode, le thiocolchicoside et la lécithine sont mélangés dans un mortier pendant 10 minutes. Le mélange homogène obtenu, ayant la consistance d'une pâte, est mélangé avec le mannitol. La poudre obtenue est tamisée à travers des tamis de 250 µm.
Selon une deuxième méthode, le thiocolchicoside et la lécithine sont dissous dans 6 ml d'un mélange chloroforme /méthanol : 1 / 1. Le mannitol est granulé dans un mortier en ajoutant successivement des portions de 0,4 ml de solution organique précédemment obtenue. La poudre obtenue est tamisée à travers des tamis de 250 µm.
Selon une troisième et dernière méthode, la poudre est produite par séchage au moyen d'un atomiseur (Mini Spray Dryer B191 BUCHI) d'une émulsion obtenue en mélangeant, à 40°C environ, une solution de lécithine dans de l'éthanol (80 ml) avec une solution aqueuse (1000 ml) de mannitol et de thiocolchicoside. Les conditions opératoires sont : une température de l'air à l'entrée de 130°C, une température de l'air en sortie de 53°C et une vitesse d'alimentation de 7,5 ml/min.

### Légende des figures :

Les essais qu'illustrent les figures suivantes sont réalisés sur les poudres nasales décrites aux exemples 1 et 2.

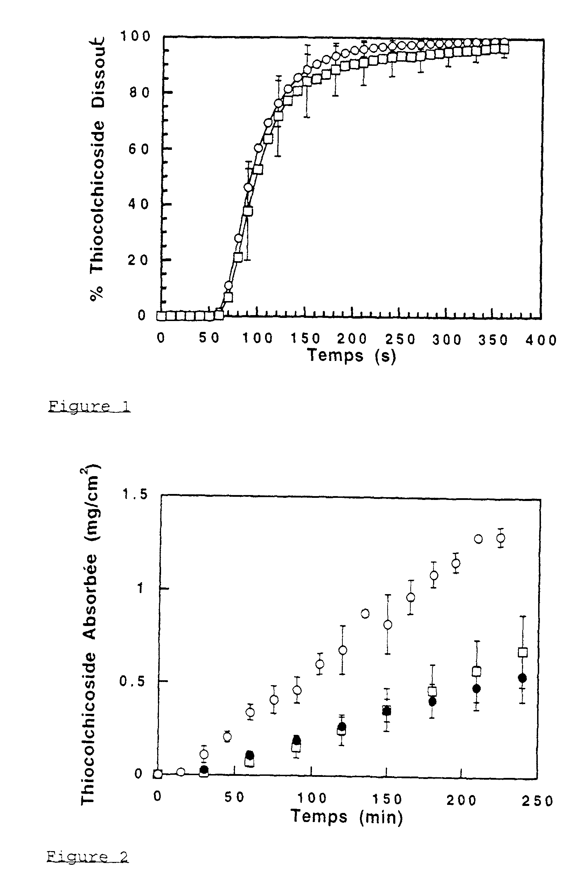

La figure 1 représente le profil de dissolution, exprimé en pourcentage, de la poudre nasale à libération immédiate (○) et de la poudre nasale à libération prolongée (□) (valeur moyenne ± sem ; n=3).
La figure 1 illustre les profils de dissolution des deux compositions pharmaceutiques à libération immédiate et à libération prolongée, caractérisées par des vitesses de pénétration différentes. Un appareil de dissolution à volume constant, comprenant un bain thermostaté à 37°C, dans lequel sont immergés les vases de dissolution est utilisé. Les vases de dissolution sont reliés à un spectrophotomètre UV-VIS à travers une pompe péristaltique. La dissolution est conduite dans 1000 ml d'eau à 37°C, avec une agitation de 100 tour/minute en utilisant la méthode de la palette rotative de la USP XIII. Le dosage du thiocolchicoside dissout est réalisé par lecture spectrophotométrique à 370 nm.

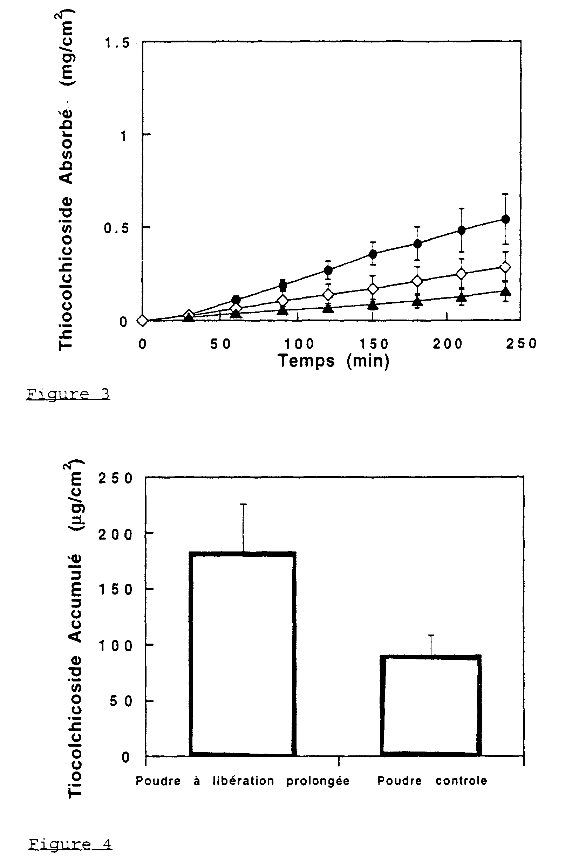

La figure 2 représente la quantité de thiocolchicoside absorbée, par unité de surface de muqueuse, *in vitro* à travers la muqueuse de lapin, de la poudre à libération immédiate (○), de la "poudre de contrôle" (●) et d'une solution saturée de poudre à libération immédiate (□) (valeur moyenne ± sem ; n≥6).
La figure 2 compare les profils de pénétration du thiocolchicoside obtenus *in vitro* à travers de la muqueuse nasale de lapin. Une poudre nasale à libération immédiate est comparée à la fois à une "poudre de contrôle", obtenue en mélangeant du thiocolchicoside et du mannitol (1 : 9) et à une solution saturée en poudre nasale à libération immédiate. Les essais de pénétration à travers la muqueuse nasale de lapin sont conduits selon la méthode expérimentale décrite ci-après.
Les échantillons de muqueuse nasale de lapin sont prélevés après décollement de la muqueuse de la cloison cartilagineuse des fosses nasales. Des. cellules de diffusion verticales en verre (Franz), d'une surface de 0,196 cm², sont utilisées. L'échantillon de muqueuse, prélevé quelques heures après l'abattage du lapin, est monté horizontalement entre la compartiment donneur et récepteur, la muqueuse étant tournée vers le compartiment donneur. Le compartiment donneur est rempli de 3,8 ml de solution physiologique dégazée. La poudre nasale est placée dans le compartiment donneur. Le compartiment donneur est, par la suite, recouvert d'un papier filtre imbibé de solution physiologique et fermé avec de la parapellicule. Les essais sont conduits à température ambiante et poursuivis pendant 4 heures. On prélève 0,2 ml de la solution réceptrice à des intervalles réguliers et on les remplace par une solution physiologique dégazée fraîche. Les échantillons prélevés sont analysés par HPLC.

La figure 3 représente la quantité de thiocolchicoside absorbée, par unité de surface de muqueuse *in vitro* à travers la muqueuse de lapin, d'une poudre à libération prolongée contenant 10% de thiocolchicoside et 6,7% de lécithine (◇) d'une poudre à libération prolongée contenant 6% de thiocolchicoside et 11,8% de lécithine (▲) et de la "poudre de contrôle" (●) (valeur moyenne ± sem ; n≥6).
La figure 3 compare les profils de pénétration de thiocolchicoside obtenus *in vitro* à travers de la muqueuse nasale de lapin de deux types de poudre à libération prolongée et d'une "poudre de contrôle", obtenue en mélangeant du thiocolchicoside avec du mannitol (1 : 9).

La figure 4 représente la quantité de thiocolchicoside accumulée, par unité de volume de muqueuse, dans la muqueuse nasale de lapin, à partir d'une poudre à libération prolongée contenant 10% de thiocolchicoside et 6,7% de lécithine et de "poudre contrôle" (valeur moyenne ± sem ; n≥6).
La figure 4 compare l'accumulation dans la muqueuse nasale de lapin d'une poudre nasale à libération prolongée et de la "poudre de contrôle" décrite ci-dessus. L'accumulation de thiocolchicoside sans la muqueuse nasale a été mesurée en extrayant le thiocolchicoside selon la méthode expérimentale décrite ci-après.
Au terme de l'essai de pénétration, le compartiment donneur est lavé avec de l'eau distillée, la muqueuse est prélevée de la cellule puis broyée pendant 5 minutes dans un homogénéisateur (potter) avec 1 ml d'eau distillée. L'homogénat obtenu est transféré dans un ballon taré de 25 ml avec 5 ml d'eau distillée et placé sous ultra-sons pendant 30 minutes. On ajoute ensuite du laurisulfate de sodium (1%) puis, successivement, de l'acide perchlorique à 10% (5 ml) et de l'eau distillée jusqu'à 25 ml. Le ballon est placé pendant 10 minutes sous ultra-sons. La solution est filtrée et analysée par HPLC.

Les résultats illustrés par les figures 1 à 4 s'interprètent comme suit :

Les profils de dissolution des deux types de compositions sont similaires (Figure 1). Ceci montre que le ralentissement de la pénétration du thiocolchicoside à travers la muqueuse nasale de lapin, observé pendant les essais, n'est pas à attribuer à un ralentissement de la dissolution de la poudre, mais à la formation d'un réservoir dans la muqueuse, dont le principe actif est libéré lentement.

Le thiocolchicoside présente de très bonnes caractéristiques de pénétration à travers la muqueuse nasale de lapin avec un coefficient de perméabilité de 2,8.10⁻⁶ ± 0,2.10⁻⁶ cm.s⁻¹. Ce coefficient de perméabilité est calculé d'après l'équation (2.10) "Controlled release of biologically active agents", page 24 (Ed. R.W.Baker, John Willey & Sons, New York, 1987).

La β-cyclodextrine favorise l'absorption du thiocolchicoside à travers la muqueuse nasale du lapin (figure 2) : La vitesse d'absorption du thiocolchicoside de la composition à libération immédiate (9,04.10⁻⁵ ± 1.49.10⁻⁵ mg.cm⁻².s⁻¹) est supérieure à celle de la poudre de contrôle (3,43.10⁻⁵ ± 0,65.10⁻⁵ mg. cm⁻². s⁻¹).

La figure 2 illustre l'intérêt d'une formulation à l'état de poudre par rapport à une formulation liquide. En effet, la vitesse de pénétration et la quantité d'absorption du thiocolchicoside sont nettement supérieures pour la composition à libération immédiate que pour la solution saturée en thiocolchicoside, obtenue à partir de la même composition à libération immédiate.

La figure 3 montre que la composition à libération prolongée, de part une quantité d'absorption et une vitesse de pénétration inférieures à celles de la poudre contrôle, est en mesure de maintenir la libération sur une période plus longue.

L'accumulation de thiocolchicoside obtenue dans la muqueuse grâce à la composition à libération prolongée (figure 4), est caractéristique d'un transport prolongé, au travers de la muqueuse nasale, du principe actif *in* vivo.

## Revendications

1. Composition pharmaceutique administrable par voie nasale comprenant de thiocolchicoside, **caractérisée en ce qu'**elle se présente sous forme de poudre.

2. Composition pharmaceutique selon la revendication 1, **caractérisée en ce que** la quantité de thiocolchicoside est comprise entre 2 et 10 mg par unité, de préférence entre 2 et 8 mg.

3. Composition pharmaceutique selon l'une quelconque des revendications 1 ou 2, pour la libération immédiate de thiocolchicoside, **caractérisée en ce qu'**elle comprend en plus du thiocolchicoside, une cyclodextrine et éventuellement un ou plusieurs autres excipients pharmaceutiquement acceptable.

4. Composition pharmaceutique selon la revendication 3, **caractérisée en ce qu'**elle comprend, en masse, 5 à 40% de thiocolchicoside, 30 à 90% de cyclodextrine et 0 à 65% d'un ou plusieurs autres excipients pharmaceutiquement acceptables.

5. Composition pharmaceutique selon la revendication 3 ou 4, **caractérisée en ce que** la cyclodextrine est la β-cyclodextrine.

6. Composition pharmaceutique selon l'une quelconque des revendications 1 ou 2, pour la libération prolongée de thiocolchicoside, **caractérisée en ce qu'**elle comprend en plus du thiocolchicoside, un phospholipide et un ou plusieurs excipients pharmaceutiquement acceptables.

7. Composition pharmaceutique selon la revendication 6, **caractérisée en ce qu'**elle contient, en masse, 5 à 40% de thiocolchicoside, 6 à 15% de phospholipide et 45 à 89% d'un ou plusieurs autres excipients pharmaceutiquement acceptables.

8. Composition pharmaceutique selon la revendication 6 ou 7, **caractérisée en ce que** le phospholipide est la lécithine.

9. Procédé de préparation d'une composition pharmaceutique selon l'une quelconque des revendications 1 à 8, **caractérisé en ce qu'**il consiste à mélanger les composants à sec ou dans un solvant, dans un mortier, puis à tamiser le mélange obtenu ou bien à utiliser un atomiseur.

10. Utilisation du thiocolchicoside avec un ou plusieurs excipients pharmaceutiquement acceptables, pour la fabrication d'un médicament sous forme de poudre administrable par voie nasale, pour le traitement d'appoint des contractures musculaires douloureuses.

11. Composition pharmaceutique selon l'une quelconque des revendications 1 à 5 pour la libération immédiate de thiocolchicoside **caractérisée en ce qu'**elle contient 2 g de thiocolchicoside et 18 g de β-cyclodextrine.

12. Composition pharmaceutique selon l'une quelconque des revendications 1 à 2 et 6 à 8 pour la libération prolongée de thiocolchicoside **caractérisée** en ce quelle contient 2 g de thiocolchicoside, 16, 66 g de mannitol et 1, 34 g de lécithine.

## Patentansprüche

1. Pharmazeutische Zubereitung, die auf nasalem Wege verabreicht werden kann, enthaltend Thiocolchicosid, **dadurch gekennzeichnet, daß** sie in Form eines Pulvers vorliegt.

2. Pharmazeutische Zubereitung nach Anspruch 1, **dadurch gekennzeichnet, daß** die Thiocolchicosid-Menge zwischen 2 und 10 mg pro Einheit, vorzugsweise zwischen 2 und 8 mg liegt.

3. Pharmazeutische Zubereitung nach einem der Ansprüche 1 oder 2 für die sofortige Freisetzung von Thiocolchicosid, **dadurch gekennzeichnet, daß** sie zusätzlich zu Thiocolchicosid ein Cyclodextrin und gegebenenfalls eines oder mehrere andere pharmazeutisch annehmbaren Trägermaterialien umfaßt.

4. Pharmazeutische Zubereitung nach Anspruch 3, **dadurch gekennzeichnet, daß** sie 5 bis 40 Massen-% Thiocolchicosid, 30 bis 90 Massen-% Cyclodextrin und 0 bis 65 Massen-% eines oder mehrerer anderer pharmazeutisch annehmbarer Trägermaterialien umfaßt.

5. Pharmazeutische Zubereitung nach Anspruch 3 oder 4, **dadurch gekennzeichnet, daß** das Cyclodextrin β-Cyclodextrin ist.

6. Pharmazeutische Zubereitung nach einem der Ansprüche 1 oder 2 für die verlängerte Freisetzung von Thiocolchicosid, **dadurch gekennzeichnet, daß** sie zusätzlich zu Thiocolchicosid ein Phospholipid und ein oder mehrere pharmazeutisch annehmbare Trägermaterialien umfaßt.

7. Pharmazeutische Zubereitung nach Anspruch 6, **dadurch gekennzeichnet, daß** sie 5 bis 40 Massen-% Thiocolchicosid, 6 bis 15 Massen-% Phospholipid und 45 bis 89 Massen-% eines oder mehrerer anderer pharmazeutisch annehmbarer Trägermaterialien umfaßt.

8. Pharmazeutische Zubereitung nach Anspruch 6 oder 7, **dadurch gekennzeichnet, daß** das Phospholipid Lecithin ist.

9. Verfahren zur Herstellung einer pharmazeutischen Zubereitung nach einem der Ansprüche 1 bis 8, **dadurch gekennzeichnet, daß** es darin besteht, die Bestandteile trocken oder in einem Lösungsmittel in einem Mörser zu vermischen und dann die erhaltene Mischung zu sieben oder eine Sprüheinrichtung zu verwenden.

10. Verwendung von Thiocolchicosid zusammen mit einem oder mehreren pharmazeutisch annehmbaren Trägermaterialien für die Herstellung eines Arzneimittels in Form eines auf nasalem Wege verabreichbaren Pulvers zur ergänzenden Behandlung von schmerzhaften Muskelkontrakturen.

11. Pharmazeutische Zubereitung nach einem der Ansprüche 1 bis 5 für die sofortige Freisetzung von Thiocolchicosid, **dadurch gekennzeichnet, daß** sie 2 g Thiocolchicosid und 18 g β-Cyclodextrin enthält.

12. Pharmazeutische Zubereitung nach einem der Ansprüche 1 bis 2 und 6 bis 8 für die verlängerter Freisetzung von Thiocolchicosid, **dadurch gekennzeichnet, daß** sie 2 g Thiocolchicosid, 16,66 g Mannit und 1,34 g Lecithin enthält.

## Claims

1. Pharmaceutical thiocolchicoside-containing composition which can be administered by the nasal route, **characterized in that** it is in the form of a powder.

2. Pharmaceutical composition according to Claim 1, **characterized in that** the quantity of thiocolchicoside is between 2 and 10 mg per unit, preferably between 2 and 8 mg.

3. Pharmaceutical composition according to either of Claims 1 and 2, for the immediate release of thiocolchicoside, **characterized in that** it comprises, in addition to thiocolchicoside, a cyclodextrin and optionally one or more other pharmaceutically acceptable excipients.

4. Pharmaceutical composition according to Claim 3, **characterized in that** it comprises, by mass, 5 to 40% of thiocolchicoside, 30 to 90% of cyclodextrin and 0 to 65% of one or more other pharmaceutically acceptable excipients.

5. Pharmaceutical composition according to Claim 3 or 4, **characterized in that** the cyclodextrin is β-cyclodextrin.

6. Pharmaceutical composition according to either of Claims 1 and 2, for the prolonged release of thiocolchicoside, **characterized in that** it comprises, in addition to thiocolchicoside, a phospholipid and one or more pharmaceutically acceptable excipients.

7. Pharmaceutical composition according to Claim 6, **characterized in that** it contains, by mass, 5 to 40% of thiocolchicoside, 6 to 15% of phospholipid and 45 to 89% of one or more other pharmaceutically acceptable excipients.

8. Pharmaceutical composition according to Claim 6 or 7, **characterized in that** the phospholipid is lecithin.

9. Method for preparing a pharmaceutical composition according to any one of Claims 1 to 8, **characterized in that** it consists in mixing the components in the dry state or in a solvent, in a mortar, and then in sieving the mixture obtained or alternatively in using a spray-dryer.

10. Use of thiocolchicoside with one or more pharmaceutically acceptable excipients for the manufacture of a medicament in the form of a powder which can be administered by the nasal route, for the adjunctive treatment of painful muscular contractures.

11. Pharmaceutical composition according to any one of Claims 1 to 5 for the immediate release of thiocolchicoside, **characterized in that** it contains 2 g of thiocolchicoside and 18 g of β-cyclodextrin.

12. Pharmaceutical composition according to any one of Claims 1 to 2 and 6 to 8 for the prolonged release of thiocolchicoside, **characterized in that** it contains 2 g of thiocolchicoside, 16.66 g of mannitol and 1.34 g of lecithin.
